## Europäisches Patentamt
## European Patent Office
## Office européen des brevets

(19)

(11) Numéro de publication: **0 014 011**
**B1**

(12) **FASCICULE DE BREVET EUROPEEN**

(45) Date de publication du fascicule du brevet: **09.02.83**

(51) Int. Cl.³: **C 12 P 7/58, C 12 N 9/04**

(21) Numéro de dépôt: **80200030.7**

(22) Date de dépôt: **14.01.80**

(54) **Procédé pour la fabrication d'acides aldoniques par voie enzymatique.**

(30) Priorité: **22.01.79 FR 7901861**

(43) Date de publication de la demande:
**06.08.80 Bulletin 80/16**

(45) Mention de la délivrance du brevet:
**09.02.83 Bulletin 83/6**

(84) Etats contractants désignés:
**AT BE CH DE FR GB IT LU NL SE**

(56) Documents cités:
**DE - A - 563 758**
**FR - A - 1 590 031**
**FR - A - 2 049 969**
**FR - A - 2 177 931**
**GB - A - 1 006 903**
**US - A - 1 896 811**
**US - A - 2 006 086**
**US - A - 2 351 500**
**US - A - 2 651 592**

(73) Titulaire: **SOLVAY & Cie (Société Anonyme)**
**Rue du Prince Albert, 33**
**B-1050 Bruxelles (BE)**

(72) Inventeur: **Coppens, Guillaume**
**Avenue Louis Jasmin, 89**
**B-1150 Bruxelles (BE)**

(74) Mandataire: **Eischen, Roland**
**Solvay & Cie Département de la Propriété**
**Industrielle Rue de Ransbeek 310**
**B-1120 Bruxelles (BE)**

Courier Press, Leamington Spa, England.

(56) Documents cités:

CHEMICAL ABSTRACTS, vol. 28, no. 12, 20 Juin 1934, abrégé 3831(5) Columbus, Ohio, U.S.A. O. E. MAY et al.: "Gluconic acid".

CHIMIA, vol. 29, no. 3, mars 1975, pages 123—131 CH VON. V. KRASNOBAJEW et al.: "Verwendungsmöglichkeiten von PAG-Copolymerimmobilisierten Enzymen in der Stärkeindustrie"

BIOTECHNOLOGY AND BIOENGINEERING, vol. XIX, 19(2), 1977, pages 185—198, John Wiley & Sons, Inc. Y. K. CHO et al.: "Glucoamylase and glucose oxidase preparations and their combined application for conversion of maltose to gluconic acid

**0014011**

Procédé pour la fabrication d'acides aldoniques par voie enzymatique

La présente invention concerne un procédé pour la fabrication par voie enzymatique d'acides aldoniques par oxydation des oses correspondants.

Il est connu d'oxyder les oses en les acides aldoniques correspondants par voie enzymatique en mettant en oeuvre les enzymes spécifiques de ces réactions appelées généralement oxydases. Ainsi, on oxyde le glucose en acide gluconique à l'intervention de la glucose oxydase. Par ailleurs, diverses techniques destinées à rendre cette réaction plus intéressante d'un point de vue industriel sont connues également. Ainsi, on a déjà proposé dans le document FR—A—2177931 (BOEHRINGER MANNHEIM GmbH) de convertir le glucose en acide gluconique par oxydation au moyen d'oxygène en solution aqueuse en présence d'un catalyseur contenant de la glucoseoxydase et de la catalase fixées conjointement sur un support. On a également déjà proposé dans le document FR—A—2049969 (HAYASHIBARA COMPANY) d'obtenir des acides aldoniques par oxydation enzymatique ou fermentive d'hydrolysats d'amidon.

Bien que la réaction d'oxydation du glucose en acide gluconique à l'intervention de la glucose oxydase soit très sélective, les techniques connues présentent cependant l'inconvénient de donner de faibles taux de transformation par unité d'enzyme mise en oeuvre, ce qui conduit à une très faible productivité et rend le procédé économiquement peu rentable.

La présente invention vise à fournir un procédé pour la fabrication d'acides aldoniques par oxydation des oses correspondants qui ne présente par les inconvénients des procédés conventionnels et qui permet notamment d'améliorer sensiblement les productivités.

Elle concerne à cet effet un procédé pour la fabrication d'acides aldoniques par oxydation par voie enzymatique des oses correspondants au moyen d'oxygène selon lequel on maintient dans le mélange réactionnel un rapport molaire oxygène sur ose supérieur à 0,1 sous une pression partielle en oxygène d'au moins 0,5 bar.

De préférence on utilise un rapport molaire oxygène sur ose supérieur à 0,2. De bons résultats ont été obtenus quand ce rapport molaire est compris entre 0,2 et 50. Des rapports molaires supérieurs à 50 conviennent également mais présentent moins d'intérêt d'un point de vue économique.

L'oxygène servant à l'oxydation de l'ose en l'acide aldonique correspondant peut être mis en oeuvre sous différentes formes. En général, on dissout de l'oxygène gazeux dans le mélange réactionnel. L'oxygène peut être mis en oeuvre sous forme d'oxygène pur ou de mélanges d'oxygène avec des inertes.

En général, on utilise un mélange gazeux contenant essentiellement de l'oxygène, de préférence à raison d'au moins 90% en poids.

La quantité d'oxygène dissoute dans le milieu est en général supérieure à 20 mg/l. Avantageusement on utilise des conditions telles que la quantité d'oxygène dissoute dans le milieu réactionnel est comprise entre 40 et 10000 mg/l.

Les pressions partielles en oxygène sont, de préférence comprises entre environ 1 et 100 bars. De bons résultats ont été obtenus en utilisant des pressions de 3 à 80 bars.

La présente invention s'applique à l'oxydation de divers types d'oses. On peut ainsi oxyder du glucose, du galactose, et du lactose. D'excellents résultats ont été obtenus en appliquant le procédé de l'invention à l'oxydation du glucose en acide gluconique.

Le procédé selon l'invention peut s'appliquer à l'oxydation d'oses purs ou de mélanges d'oses. Dans ce cas, on peut sélectivement oxyder un des oses grâce à l'intervention de l'enzyme spécifique de la réaction. Ainsi dans le cas du glucose on peut appliquer l'invention à l'oxydation sélective du glucose contenu dans des mélanges contenant en outre d'autres sucres tels que le fructose, le galactose, les di-, tri- et oligosaccharides en général (maltose, cellobiose, raffinose, etc).

L'oxydation des oses en acides aldoniques correspondants se fait par voie enzymatique à l'intervention des oxydases spécifiques des réactions en cause. Les enzymes peuvent se présenter sous diverses formes, soit sous forme d'enzymes libres, soit sous forme d'enzymes immobilisées soit encore sous forme de cellules les contenant ce qui est le cas par exemple des microorganismes. De bons résultats ont été obtenus lorsqu'on met en ouvre des enzymes immobilisées.

Toutes les méthodes d'immobilisation connues en elles-même, peuvent être utilisées. Diverses techniques pour ce faire ont été proposées dans le livre de O. Zaborsky, Immobilized Enzymes, CRC Press, 1974. On peut ainsi fixer les enzymes notamment par adsorption physique sur un support, par piégeage dans des matrices, inclusion ou microencapsulation, par formation d'une liaison covalente entre le support et l'enzyme ou par coréticulation. Une technique particulièrement adéquate a fait l'objet de la demande de brevet n° 79.01054 déposée en France le 12.1.1979 par la Demanderesse (FR—A—2 446 317 publicé le 8.8.80).

La quantité d'enzyme mise en oeuvre peut varier largement. Elle dépend de la nature de l'enzyme, de la qualité commerciale utilisée et également de la forme sous laquelle elle est mise en oeuvre (immobilisée ou non). Dans le cas de l'oxydation du glucose, on utilise le glucose oxydase en général à raison de environ 0,1 à 10.000 unités internationales par gramme de glucose à transformer. De préférence, ces quantités sont comprises entre 1 et 1000 unités internationales par gramme de glucose

3

**0014011**

à transformer.

Les réactions d'oxydation des oses en acides aldoniques correspondants sont en général réalisées en milieu solvant. Divers solvants peuvent être utilisés à cette fin. En général on utilise comme solvant de l'eau seule ou en mélange avec d'autres solvants inertes vis-à-vis de la réactions tels que des alcools. De bons résultats ont été obtenus en utilisant l'eau comme solvant.

Outre les réactifs ainsi que les autres constituants éventuels présents dans l'ose de départ, les produits de la réaction, les solvants et l'enzyme, le mélange réactionnel peut également contenir d'autres constituants. Parmi ceux-ci on peut citer des agents régulateurs de pH, des agents pour la séparation ou la neutralisation des acides aldoniques formés, des agents bactéricides ou fongicides tels que l'oxyde d'éthylène (afin de contrôler une éventuelle pollution bactérienne), et des additifs permettant d'améliorer l'activité de l'enzyme. On peut aussi, s'il y a lieu, ajouter des agents pour l'élimination du peroxyde d'hydrogène formé. La catalase convient bien à cet effet.

Le pH est habituellement maintenu dans certaines limites, qui sont choisies en fonction de la nature de l'enzyme spécifique de la réaction afin d'éviter une dégradation ou une inactivation de l'enzyme. Ainsi, dans le cas de l'oxydation du glucose en acide gluconique, on maintient en général le pH à une valeur comprise entre 4,2 et 8. Si l'enzyme est immobilisée on préfère utiliser des pH compris entre 5,0 et 8. Si, par contre, on met en oeuvre une enzyme libre on utilise de préférence des pH entre 4,2 à 7.

Pour maintenir le pH à la valeur souhaitée, on peut utiliser les diverses techniques connues. Ainsi, on peut ajouter au milieu des tampons. On peut également neutraliser l'acide aldonique au fur et à mesure de sa formation par addition d'une base. Parmi celles utilisables, on peut citer les hydroxydes et carbonates alcalins et alcalino-terreux tels que l'hydroxyde de sodium ou de potassium, et le carbonate de sodium ou de calcium, ainsi que les bases organiques. De bons résultats ont été obtenus en neutralisant l'acide aldonique au moyen d'hydroxyde ou de carbonate de sodium. On peut enfin retirer en continu l'acide gluconique du mélange réactionnel par diverses techniques connues en elles-mêmes telles que l'électrodialyse, le mise en ouvre de résines échangeuses d'ions, ou la précipitation.

La température de réaction est choisie en fonction de la nature de l'enzyme spécifique mise en oeuvre. On utilise de préférence les températures de fonctionnement optimal de l'enzyme. Dans le cas de l'oxidation du glucose en acide gluconique à l'intervention de glucose oxydase on utilise en général des températures comprises entre 0 et 60°C, le plus souvent entre 0 et 50°C. De bons résultats ont été obtenus en utilisant des températures comprises entre 0 et 35°C.

Le procédé selon l'invention peut être réalisé en continu ou en discontinu. Industriellement, on utilise en général des réacteurs fonctionnant en continu. Ces réacteurs peuvent être de divers types connus en eux-mêmes. Ainsi, on peut opérer dans des réacteurs mélangeurs ou dans des réacteurs tubulaires dits "réacteurs méthodiques" ou "réacteurs intégraux". On peut utiliser un réacteur unique on plusieurs réacteurs disposés en série ou en parallèle. Industriellement, quand on désire opérer en continu il est avantageux de disposer de plusieurs réacteurs de manière à pouvoir renouveler la charge d'enzyme dans un réacteur sans interrompre l'ensemble de la production.

Les enzymes, lorsqu'elles sont mises en oeuvre sous forme immobilisée peuvent être disposées dans les réacteurs sous forme de lit fixe, de lit fluidisé ou encore de lit mobile ou de lit turbulent. Les enzymes immobilisées peuvent être complètement immergées dans le mélange réactionnel liquide, ou encore on peut faire ruisseler ce dernier sur elles.

Lorsque les enzymes sont mises en oeuvre sous forme libre, on utilise en général des réacteurs mélangeurs. Ces réacteurs s'ils fonctionnent en continu sont en général pourvus de dispositifs pour éviter que l'enzyme ne quitte le réacteur au cours des prélèvements de production. Ces dispositifs peuvent être des filtres d'ultrafiltration. Après réaction les enzymes peuvent être séparées du mélange réactionnel selon diverses techniques connues en elles-mêmes telles que décantation, filtration.

L'introduction des réactifs peut se faire directement dans le réacteur contenant l'enzyme. On peut également préparer la solution contenant l'ose à oxyder et l'oxygène dans des saturateurs ou mélangeurs-saturateurs distincts du réacteur. D'autres techniques peuvent également être envisagées.

Les matériaux utilisés pour la construction des appareils nécessaires à la réalisation du procédé de l'invention sont de natures diverses. En général on utilise des matériaux résistant à la corrosion et, s'il y a lieu, à la pression. On peut ainsi utiliser des réacteurs en tantale, en inconel ou des réacteurs émaillés ou chemisés au moyen d'une substance résistant à la corrosion.

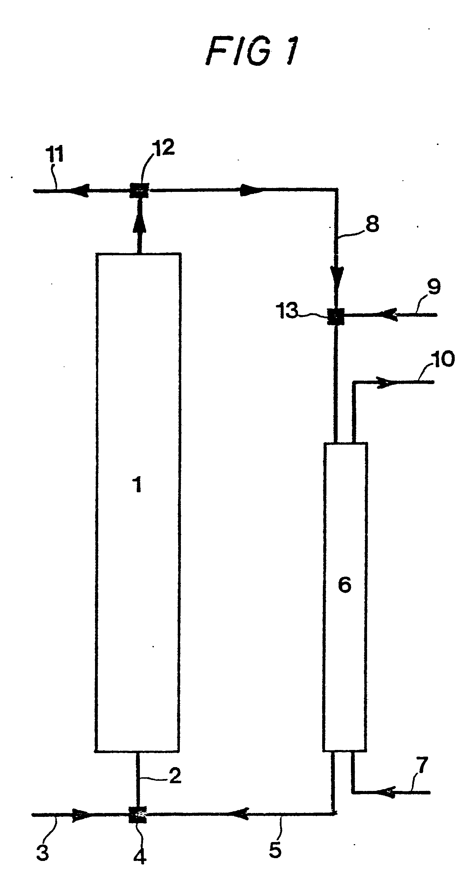

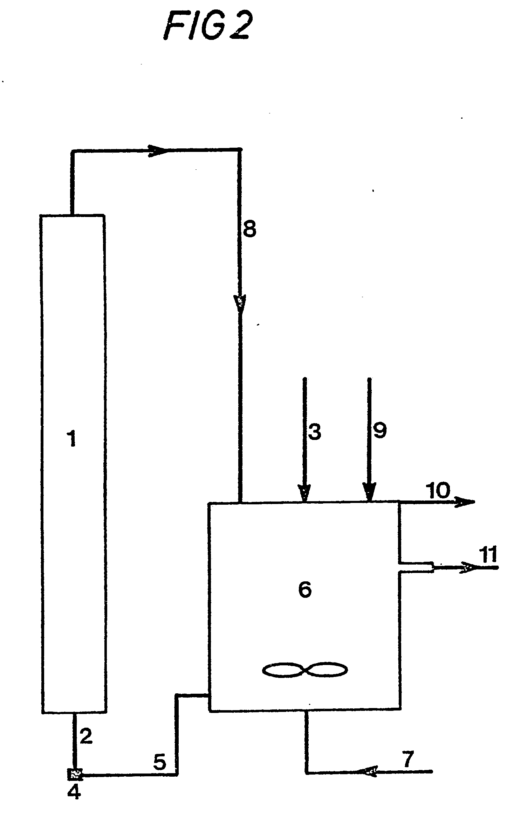

Le procédé selon l'invention peut être réalisé dans des appareils tels que ceux schématisés aux figures 1 et 2 en annexe qui représentent des modes de réalisation du procédé selon l'invention.

La figure 1 représente un réacteur 1 dans lequel on dispose des enzymes immobilisées. Le réacteur est alimenté par la voie 2 en un mélange réactionnel contenant l'ose, le solvant et l'oxygène. Ce mélange est issu du mélangeur 4 qui est alimenté en ose par la voie 3 et en une solution saturée en oxygène par la voie 5.

La solution saturée en oxygène est obtenue dans le saturateur 6 par barbotage d'oxygène introduit par la voie 7 dans la solution navette issue du réacteur 1 et amenée par la voie 8. L'excès d'oxygène quitte le saturateur 6 par la voie 10.

La solution navette issue de réacteur est neutralisée en 13 par addition d'une base par la voie 9.

On prélève en continu en 12 par la voie 11 une partie de la solution navette qui constitue la

4

production.

La figure 2 représente un appareillage similaire à celui de la figure 1 dans lequel l'ose est introduite directement par la voie 3 dans un mélangeur saturateur 6, ainsi que la base neutralisante qui est introduite par la voie 9. L'excès d'oxygène quitte le mélangeur saturateur par la voie 9 et la production est recueillie par débordement par la voie 11.

Selon les procédés schématisés aux figures 1 et 2, on fait circuler en navette une solution qui contient essentiellement le solvant, l'ose non transformée et l'acide aldonique partiellement neutralisé. La proportion de ce dernier dans la solution peut varier dans de larges limites. En général, on opère dans des conditions proches de la concentration à saturation. Ainsi lors de l'oxydation du glucose en acide gluconique à l'intervention de la glucose oxydase, si on utilise comme agent neutralisant un dérivé sodique, la concentration de gluconate de sodium dans la solution peut atteindre 500 g. $l^{-1}$ selon la température et est en général comprise entre 50 et 450 g. $l^{-1}$.

Le procédé de l'invention convient particulièrement bien pour transformer sélectivement un ose particulier parmi un mélange d'oses. Ainsi on peut avantageusement appliquer le procédé de l'invention à la transformation sélective du glucose dans des mélanges de sucres tels que par exemple les mélanges de glucose et de fructose obtenus par hydrolyse du saccharose ou par isomérisation du glucose ou de mélanges de glucose et de fructose obtenus à partir de polysaccharides riches en fructose tels que l'inuline et ses dérivés (dans ces cas, la concentration en acide gluconique — ou gluconate — peut varier de 1 à 200 g/l). Cette technique suivie d'une séparation de l'acide gluconique ou de son sel, permet de séparer le fructose pur. Ce produit est quant à lui utilisable pour des préparations diététiques, pharmaceutiques ou dans des aliments pour diabétiques.

Le procédé de l'invention présente l'avantage de permettre l'obtention de solutions particulièrement concentrées en acides aldoniques (ou en leurs sels correspondants), ce qui simplifie particulièrement les étapes de séparation ultérieures. En outre pour un type d'enzyme défini il permet d'obtenir des rendements en acides aldoniques par unité d'enzyme sensiblement meilleurs. Lors de la mise en oeuvre du procédé de l'invention on constate que les durées de vie des enzymes mises en oeuvre sont accrues.

Les acides aldoniques le leurs sels, obtenus selon le procédé de l'invention peuvent être utilisés pour le décapage des métaux, pour le nettoyage des bouteilles, comme agent séquestrant, pour la fabrication de produits pharmaceutiques et alimentaires.

Les examples ci-après sont donnés afin de mettre en évidence les avantages du procédé selon l'invention par rapport aux procédés conventionnels. Les essais 1, 5, 6 et 11 été réalisés à titre de comparaison et les essais 2, 3, 4, 7, 8, 9 et 10 ont été réalisés selon l'invention.

Oxydation du glucose à l'intervention de la glucose oxydase

### Exemple 1 (comparatif)

L'enzyme mise en oeuvre est une glucose oxydase commerciale ayant une activité de catalase, fournie par la firme SIGMA CHEMICAL Co et contenant 15.000 unités internationales d'enzyme par gramme. L'enzyme a été immobilisée sous forme de mousse selon la technique décrite dans l'article de G. BROUN, D. THOMAS, G. GELLF, D. DOMURADO, A. M. BERJONNEAU et C. GUILLON, Biotechnology and Bioengineering, Vol. XV p. 359—375 (1973).

Les essais ont été réalisés dans un réacteur en verre de 200 cm$^3$ pourvu d'un trop-plein et d'un agitateur et ayant un volume utile de 100 cm$^3$. Le réacteur est maintenu à une température de 25°C.

On dispose dans le réacteur 35 mg de glucose oxydase immobilisée et 100 cm$^3$ d'une solution contenant 37,8 g.$l^{-1}$ de glucose et 0,01 mol.$l^{-1}$ de tampon phosphate de pH 6,8.

On fait barboter en continu dans la solution de l'oxygène sous pression atmosphérique. La teneur en glucose de la solution est maintenue constante par addition continue d'une solution à 410 g.$l^{-1}$ de glucose. Le rapport molaire oxygène sur glucose est d'environ 0,059 pendant toute la réaction.

Le pH de la solution est maintenu constant par addition continue d'une solution à 1 mol.$l^{-1}$ d'hydroxyde de sodium saturée au préalable en oxygène à la pression atmosphérique. L'acide gluconique est ainsi neutralisé en gluconate de sodium.

On recueille en continu le trop-plein dans lequel on dose le gluconate de sodium.

Après 6,6 heures de fonctionnement on a oxydé 2,94 g de glucose, ce qui correspond à 5,6 mg de glucose transformé par unité internationale de glucose oxydase mise en oeuvre.

### Exemple 2

L'enzyme mise en oeuvre est identique à celle utilisée à l'exemple 1. L'enzyme a été immobilisée sur des granules de pierre ponce de granulométrie comprise entre 0,5 et 1 mm selon la technique décrite ci-après.

On met en solution 151,5 mg de glucose oxydase dans 54 ml d'une solution aqueuse de tampon phosphate. On prépare simultanément une solution dans 42 ml du tampon phosphate de 8,4 g d'albumine. Après dissolution complète de l'albumine on mélange les solutions et on ajoute 54 ml d'une solution tamponnée contenant 810 mg de glutaraldéhyde.

Après homogénéisation on ajoute 170 g de pierre ponce. Le mélange obtenu est mis sous vide

5

# 0014011

afin d'éliminer l'air occlus et après retour à la pression atmosphérique il est maintenu pendant environ 4 h à −35°C. Le produit est ensuite ramené à la température ambiante et lavé avec une solution de glycocolle et ensuite une solution de tampon phosphate.

Les essais ont été réalisés dans un appareillage du même type que celui présenté à la figure 2.

On dispose dans un réacteur de 300 cm³ maintenu à 25°C, la glucose oxydase immobilisée sur la pierre ponce. Dans le mélangeur on introduit une solution de glucose à 40 g.l⁻¹ à raison de 850 mg de glucose par heure. La solution est saturée par de l'oxygène pur à la pression atmosphérique. Le pH est maintenu à 7,6 par addition d'une solution aqueuse contenant 1 mol.l⁻¹ d'hydroxyde de sodium.

La navette circule dans le réacteur avec un débit de 10 l.h⁻¹. La teneur moyenne en glucose de la navette est de 800 mg.l⁻¹ ce qui correspond à un rapport molaire glucose sur oxygène dissous de 3,6.

Après 42 h on a transformé 42 g de glucose, soit 18,5 mg de glucose par unité de glucose oxydase.

### Exemple 3

L'enzyme mise en oeuvre est identique à celle utilisée à l'exemple 1. L'enzyme a été immobilisée à raison de 1,29 g sur 2,4 kg de granules de titane spongieux de granulométrie comprise entre 1 et 2 mm selon la technique décrite dans la demande de brevet précitée.

Les essais ont été réalisés dans un appareillage du même type que celui présenté à la figure 1.

On dispose dans un réacteur de 4 l maintenu à 25°C, la glucose-oxydase immobilisée sur le titane spongieux. On introduit en continu dans la navette par la voie 3 une solution de glucose à 41 g.l⁻¹ à raison de 1,3 g de glucose par heure. La solution est saturée en 6 par de l'oxygène pur à une pression de 2,45 bar. Le pH est maintenu à 6,88 par addition par la voie 9 d'une solution aqueuse contenant 1 mol.l⁻¹ d'hydroxyde de sodium.

La navette circule dans le réacteur avec un débit de 10 l.h⁻¹. La teneur moyenne en glucose de la navette est de 90 mg.l⁻¹ ce qui correspond à un rapport molaire glucose sur oxygène dissous de 0,16.

Après 750 h de fonctionnement on a transformé 980,4 g de glucose, soit 51 mg de glucose par unité de glucose oxydase.

### Exemple 4

L'enzyme mise en ouvre est identique à celle utilisée à l'exemple 1. L'enzyme a été immobilisée à raison de 1 mg sur 40 g de granules de pierre ponce selon la technique décrite à l'exemple 2.

On prépare, dans un mélangeur-saturateur métallique résistant à la pression, une solution de glucose à 1 g.l⁻¹ saturée par de l'oxygène pur sous une pression de 20,6 bar. Le pH de la solution est maintenu à 6,88 grâce à la présence d'un tampon phosphate à raison de 0,1 mole.l⁻¹.

La solution est introduite en continu, avec un débit de 40 ml.h⁻¹, dans un réacteur métallique maintenu à 25°C de 50 cm³ dans lequel est disposée l'enzyme immobilisée. Le réacteur est maintenu sous une pression d'environ 21 bar.

La teneur moyenne en glucose de la solution dans le réacteur est de 470 mg.l⁻¹ ce qui correspond à un rapport molaire glucose sur oxygène dissous d'environ 0,1.

Après 280 h on a transformé 3,63 g de glucose soit 242 mg de glucose par unité de glucose oxydase.

Les résultats des essais 1 à 4 sont repris tableau I ci-après.

### TABLEAU I

| Exemple | Quantité de glucose transformé par unité d'enzyme, mg | $\dfrac{(oxygène)}{(glucose)}$ mol./mol. | pression $O_2$, bar |
|---|---|---|---|
| 1 (comp,) | 5,6 | 0,0059 | 1,01 |
| 2 | 18,5 | 0,278 | 1,01 |
| 3 | 51 | 6,25 | 2,45 |
| 4 | 242 | 10 | 20,6 |

L'examen du tableau I, montre que grâce à la mise en oeuvre de rapports molaires oxygène:glucose supérieurs à 0,1 on accroît sensiblement la quantité de glucose transformé par unité d'enzyme. Cet accroissement est d'autant plus sensible que la pression est élevée.

### Exemple 5 (comparatif)

L'enzyme mise en oeuvre est une glucose oxydase commerciale différente de celle utilisée aux

6

exemples 1 à 4, ayant une activité de catalase, fournie par la firme SIGMA CHEMICAL Co et contenant environ 45.000 unités internationales d'enzyme par gramme.

L'enzyme a été immobilisée à raison de 8,1 g sur 2,4 kg de granules de titane spongieux selon la technique décrite dans la demande de brevet précitée.

Les essair ont été réalisés dans un appareillage du même type que celui présenté à la figure 1.

On dispose dans un réacteur de 4 l maintenu à 25°C la glucose oxydase immobilisée. On introduit en continu par la voie 3 une solution de glucose à 690 g.l$^{-1}$ à raison de 6,5 g de glucose par heure. La solution est saturée par de l'oxygène pur à une pression de 2,45 bar. Le pH est maintenu à 5,6 par addition par le voie 9 d'une solution aqueuse contenant 10 mol.l$^{-1}$ d'hydroxyde de sodium.

La navette circule dans le réacteur avec un débit de 100 l.h$^{-1}$. La teneur moyenne en glucose de la navette est de 7,7 g.l$^{-1}$ ce qui correspond à un rapport molaire glucose sur oxygène dissous de 13,7.

Après 180 h de fonctionnement on a transformé 1215 g de glucose, soit 3 mg de glucose par unité de glucose oxydase.

### Exemple 6 (comparatif)

L'enzyme mise en oeuvre est identique à celle utilisée à l'exemple 5. L'enzyme a été immobilisée à raison de 20 mg de glucose oxydase sur 16 g de titane spongieux selon la technique décrite dans la demande de brevet précitée.

Les essais ont été réalises dans un appareillage du même type que celui présenté à la figure 2.

On dispose dans un réacteur de 30 cm$^3$ maintenu à 25°C la glucose oxydase immobilisée. Dans le mélangeur on introduit une solution de glucose à 227 g.l$^{-1}$ à raison de 963 mg de glucose par heure. La solution est saturée par l'oxygène pur à la pression atmosphérique. Le pH est maintenu à 5,6 grâce à la présence dans la solution de glucose d'un tampon (phthalate de potassium + hydroxyde de sodium) à raison de 0,5 mol.l$^{-1}$.

La navette circule dans le réacteur avec un débit de 3,5 l.h$^{-1}$. La teneur moyenne en glucose de la navette est de 40 g.l$^{-1}$ ce qui correspond à un rapport molaire glucose sur oxygène dissous de 160.

Après 10 h de fonctionnement on a transformé 3,3 g de glucose soit 3,1 mg de glucose par unité d'enzyme.

### Exemple 7

L'enzyme mise en oeuvre est identique à celle utilisée à l'exemple 5. L'enzyme a été immobilisée à raison de 20 mg de glucose oxydase sur 16 g de titane spongieux selon la technique décrite dans la demande de brevet précitée.

Les essais ont été réalisés dans un appareillage du même type que celui présenté à la figure 2.

On dispose dans un réacteur de 30 cm$^3$ maintenu à 25°C la glucose oxydase immobilisée. Dans le mélangeur on introduit une solution de glucose à 24,1 g.l$^{-1}$ à raison de 100 mg de glucose par heure. La solution est saturée par de l'oxygène pur à la pression atmosphérique. Le pH est maintenu à 5,6 par addition d'une solution aqueuse contenant 1 mole.l$^{-1}$ d'hydroxyde de sodium.

La navette circule dans le réacteur avec un débit de 1,5 l.h$^{-1}$. La teneur moyenne en glucose de la navette est de 450 mg.l$^{-1}$ ce que correspond à un rapport molaire glucose sur oxygène dissous de 2.

Après 60 h, on a transformé 5,17 g de glucose soit 4,9 mg de glucose par unité d'enzyme.

### Exemple 8

L'enzyme mise en oeuvre est identique à celle utilisée à l'exemple 5. L'enzyme a été immobilisée à raison de 15 g de glucose oxydase sur 2,4 kg de granules de titane spongieux selon la technique décrite dans la demande de brevet précitée.

Les essais ont été réalisés dans un appareillage du même type que celui présenté à la figure 1.

On dispose dans un réacteur de 4 l maintenu à 25°C la glucose-oxydase immobilisée. On introduit en continu par la voie 3 une solution de glucose à 670 g.l$^{-1}$ à raison de 9 g de glucose par heure. La solution est saturée par de l'oxygène pur à une pression de 2,45 bar. Le pH est maintenu à 6,88 par addition d'une solution aqueuse contenant 10 mol.l$^{-1}$ d'hydroxyde de sodium.

La navette circule dans le réacteur avec un débit de 30 l.h$^{-1}$. La teneur moyenne en glucose de la navette est de 53,1 mg.l$^{-1}$ ce qui correspond à un rapport molaire glucose sur oxygène dissous de 0,09.

Après 550 h. on a transformé 4.530 g de glucose, soit 8,4 mg de glucose par unité de glucose oxydase. En fin d'essai, la teneur en gluconate de sodium dans la navette est de 300 g.l$^{-1}$.

### Exemple 9

L'enzyme mise en oeuvre est identique à celle utilisée à l'exemple 5. L'enzyme a été immobilisée à raison de 10 mg de glucose oxydase sur 1,6 g de titane spongieux selon la technique décrite dans la demande de brevet précitée.

On prépare dans un mélangeur-saturateur métallique résistant à la pression une solution de glucose à 1 g.l$^{-1}$ saturée par de l'oxygène pur sous une pression de 20,6 bar. Le pH de la solution est maintenu à 5,6 grâce à la présence d'un tampon phtalate (phtalate de potassium + hydroxyde de sodium) à raison de 0,1 mol.l$^{-1}$.

La solution est introduite en continu à raison de 60 mg de glucose par heure dans un réacteur métallique maintenu à 25°C d'environ 2 cm$^3$ dans lequel est disposée l'enzyme immobilisée. Le réacteur est maintenu sous une pression de 21 bar.

Une partie du mélange issu de réacteur constitue la production, tandis que l'autre partie est recyclée en continu au réacteur. Le débit de cette navette est de 2,2 l.h$^{-1}$.

La teneur moyenne en glucose de la solution dans le réacteur est de 400 mg.l$^{-1}$ ce qui correspond à un rapport molaire glucose sur oxygène dissous d'environ 0,08.

Après 600 h. on a transformé 14,7 g de glucose soit 34 mg de glucose par unité de glucose oxydase.

Les résultats des essais 5 à 9 sont repris au tableau II ci-après.

TABLEAU II

| Exemple | Quantité de glucose transformé par unité d'enzyme, mg | $\dfrac{\text{(oxygène)}}{\text{(glucose)}}$, mol./mol. | pression $O_2$, bar |
|---|---|---|---|
| 6 (comp.) | 3,1 | 0,0062 | 1 |
| 5 (comp.) | 3 | 0,073 | 2,5 |
| 7 | 4,9 | 0,5 | 1,01 |
| 8 | 8,4 | 11,1 | 2,45 |
| 9 | 34 | 12,5 | 20,6 |

L'examen du tableau II montre que grâce à la mise en oeuvre de rapports molaires oxygène:glucose supérieurs à 0,1 on accroît sensiblement la quantité de glucose transformé par unité d'enzyme. Cet accroissement est d'autant plus sensible que la pression est élevée.

Exemple 10

L'enzyme mise en oeuvre est une glucose oxydase commerciale, différente de celles utilisées aux exemple 1 à 4 et 5 à 9, ayant une activité de catalase et contenant environ 500 unités internationales d'activité enzymatique par cm$^3$ de sa solution aqueuse. L'enzyme a été immobilisée par congélation pendant 4 heures à —35°C d'un mélange contenant 4 ml de cette solution de glucose oxydase, 4 ml de glutaraldéhyde à 1,5%, 3,2 ml d'albumine bovine à 20% et 2 ml de tampon phosphate 0,02 molaire de pH 6,88. Le produit est ensuite ramené à la température ambiante, broyé et lavé avec une solution de glycocolle.

Le produit obtenu se présente sous forme d'une mousse qui est dispersée dans un réacteur en verre de 500 cm$^3$ pourvu d'un trop plein muni d'une plaque frittée, pour éviter les pertes en enzyme immobilisée. Ensuite on introduit dans le réacteur 245 cm$^3$ d'une solution ayant une concentration en glucose de 10 g/l et un pH de 5,6. Cette concentration et ce pH sont maintenus constants tout au long de l'essais par l'introduction en continu de glucose sous forme d'une solution de 100 g/l dans l'eau et d'une solution d'hydroxyde de sodium à 1 mol/l.

Le tout est maintenu à 5°C et on fait barboter en continu de l'oxygène pur sous pression atmosphérique. Le rapport molaire oxygène sur glucose est d'environ 30 pendant toute la réaction.

On recueille en continu le trop plein dans lequel on dose le gluconate de sodium formé.

Après 300 heures de fonctionnement on a oxydé 120 g de glucose, ce qui correspond à 60 mg de glucose transformé par unité internationale de glucose oxydase mise en oeuvre.

Oxydation de mélanges de glucose et de fructose

Exemple 11

L'enzyme mise en oeuvre est une glucose oxydase commerciale ayant une activité de catalase, fournie par la firme SIGMA CHEMICAL Co et contenant environ 45.000 unités internationales d'enzyme par gramme.

L'enzyme a été immobilisée à raison de 20 mg sur 16 g de granules de titane spongieux de granulométrie comprise entre 1 et 2 mm selon la technique ci-après.

On met en solution 20 mg de glucose oxydase dans 4 ml d'une solution aqueuse de tampon phosphate à pH 6,88.

On prépare simultanément une solution dans 3,2 ml du tampon phosphate de 640 mg d'albumine.

Après dissolution complète de l'albumine on mélange les solutions et on ajoute 4 ml d'une solution tamponnée contenant 60 mg de glutaraldéhyde.

**0014011**

Après homogénéisation on ajoute le titane spongieux. Le mélange obtenu est mis sous vide afin d'éliminer l'air occlus et après retour à la pression atmosphérique il est maintenu pendant environ 4 h à —35°C. Le produit est ensuite ramené à la température ambiante et lavé avec une solution de glycocolle et ensuite une solution de tampon phosphate.

Les essais ont été réalisés dans un appareillage du même type que celui présenté à la figure 1.

On dispose dans un réacteur de 30 cm³ maintenu à 25°C, la glucose oxydase immobilisée sur le titane spongieux. On introduit en continu dans la navette par la voie 3 une solution contenant 25 g.l⁻¹ de glucose et 25 g.l⁻¹ de fructose à raison de 115 mg de glucose et 115 mg de fructose par heure. Simultanément on ajoute à la navette une solution contenant 1,5 g.l⁻¹ de catalase contenant 15% de glycérine, 5% de citrate de sodium, 5% d'alcool éthylique et 5% de chlorure de sodium à raison de 25.000 unités internationales d'enzyme par heure.

Le liquide qui circule en navette est saturé en 6 par de l'oxygène pur à la pression atmosphérique. Le pH est maintenu à 5,6 par addition par la voie 9 d'une solution aqueuse contenant 1 mol.l⁻¹ d'hydroxyde de sodium.

La navette circule dans le réacteur avec un débit de 1,5 l.h⁻¹. La teneur moyenne en glucose de la navette est de 730 mg.l⁻¹ ce qui correspond à un rapport molaire glucose sur oxygène dissous de 3,2.

Après 210 h de fonctionnement on a transformé 22,6 g de glucose ce qui correspond à 20,8 mg de glucose par unité de glucose oxydase.

Exemple 12 (comparatif)

L'essai est réalisé dans le même réacteur que celui utilisé à l'exemple 10 en présence des mêmes quantités d'enzyme immobilisée de la même manière.

On introduit en continu dans la navette par la voie 3 une solution contenant 250 g.l⁻ de glucose et 250 g.l⁻¹ de fructose à raison de 1 g de glucose et 1 g de fructose par heure. Simultanément on ajoute à la navette une solution contenant 1,5 g.l⁻¹ de catalase contenant 15% de glycérine, 5% de citrate de sodium, 5% d'alcool éthylique et 5% de chlorure de sodium à raison de 25.000 unités internationales d'enzyme par heure.

Le liquide qui circule en navette est saturé en 6 par de l'oxygène pur à la pression atmosphérique. Le pH est maintenu à 5,6 par addition par la voie 9 d'une solution aqueuse contenant 1 mol.l⁻¹ d'hydroxyde de sodium.

La navette circule dans le réacteur avec un débit de 1,5 l.h⁻¹. La teneur moyenne en glucose de la navette est de 25 g.l⁻¹ ce qui correspond à un rapport molaire glucose sur oxygène dissous de 110.

Après 20 h de fonctionnement on a transformé 8,85 g de glucose ce qui correspond à 8,0 mg de glucose par unité de glucose oxydase.

La comparaison des essais 10 réalisé avec un rapport molaire oxygène sur glucose de 0,31 et 11 réalisé avec un rapport molaire oxygène sur glucose de 0,0091 montre qu'on obtient respectivement 20,8 mg et 8,0 mg de glucose transformé par unité d'enzyme, c'est-à-dire qu'on accroît sensiblement la quantité de glucose transformé par unité d'enzyme on opérant selon l'invention.

**Revendications**

1. Procédé pour la fabrication d'acides aldoniques par oxydation par voie enzymatique des oses correspondants au moyen d'oxygène caractérisé en ce que l'on maintient dans le mélange réactionnel un rapport molaire oxygène sur ose supérieur à 0,1 sous une pression partielle en oxygène d'au moins 0,5 bar.

2. Procédé selon la revendication 1 caractérisé en ce que l'on maintient un rapport molaire oxygène sur ose comprise entre 0,2 et 50.

3. Procédé selon la revendication 1 ou 2 caractérisé en ce que l'on maintient la concentration en oxygène dissous entre 40 et 10000 mg/l.

4. Procédé selon l'une quelconque des revendications 1 à 3 caractérisé en ce que l'on maintient la pression en oxygène entre 1 et 100 bars.

5. Procédé selon l'une quelconque des revendications 1 à 4 caractérisé en ce que l'on catalyse la réaction par l'enzyme correspondante sous forme immobilisée.

6. Procédé selon l'une quelconque des revendications 1 à 5 appliqué à l'oxydation du glucose en acide gluconique avec l'intervention de glucose oxydase.

7. Procédé selon la revendication 6 caractérisé en ce que l'on opère la réaction à une température comprise entre 0 et 60°C.

8. Procédé selon la revendication 7 caractérisé en ce que l'on opère la réaction à un pH comprise entre 4,2 et 8.

9. Procédé selon l'une quelconque des revendications 6 à 8 appliqué à l'oxydation sélective de glucose dans des mélanges contenant du glucose et du fructose.

**Patentansprüche**

1. Verfahren zur Herstellung von Aldonsäuren durch Oxidation der entsprechenden Osen auf enzy-

9

matischem Weg mittels Sauerstoff, dadurch gekennzeichnet, dass man im Reaktionsgemisch ein Molverhältnis von Sauerstoff zu Ose von grösser als 0,1 bei einem Sauerstoff-Partialdruck von mindestens 0,5 bar aufrecht erhält.

2. Verfahren nach Anspruch 1, dadurch gekennzeichnet, dass man ein Molverhältnis von Sauerstoff zu Ose zwischen etwa 0,2 und 50 aufrecht erhält.

3. Verfahren nach Anspruch 1 oder 2, dadurch gekennzeichnet, dass man die Konzentration an gelöstem Sauerstoff zwischen 40 und 10 000 mg/l hält.

4. Verfahren nach einem der Ansprüche 1 bis 3, dadurch gekennzeichnet, dass man den Sauerstoffdruck zwischen 1 und 100 bar hält.

5. Verfahren nach einem der Ansprüche 1 bis 4, dadurch gekennzeichnet, dass man die Reaktion durch das entsprechende Enzym in immobilisierter Form katalysiert.

6. Verfahren nach einem der Ansprüche 1 bis 5, angewendet auf die Oxidation von Glucose zu Gluconsäure unter Einsatz von Glucoseoxidase.

7. Verfahren nach Anspruch 6, dadurch gekennzeichnet, dass man die Reaktion bei einer Temperatur zwischen 0 und 60°C ausführt.

8. Verfahren nach Anspruch 7, dadurch gekennzeichnet, dass man die Reaktion bei einem pH-Wert zwischen 4,2 und 8 ausführt.

9. Verfahren nach einem der Ansprüche 6 bis 8, angewendet auf die selektive Oxidation von Glucose in Glucose und Fructose enthaltenden Gemischen.

**Claims**

1. Process for the manufacture of aldonic acids by the enzymatic oxidation of the corresponding oses by means of oxygen, characterised in that a molar ratio of oxygen to ose of more than 0.1 is maintained in the reaction mixture, under an oxygen partial pressure of at least 0.5 bar.

2. Process according to Claim 1, characterised in that a molar ratio of oxygen to ose of between 0.2 and 50 is maintained.

3. Process according to Claim 1 or 2, characterised in that the dissolved oxygen concentration is kept between 40 and 10,000 mg/l.

4. Process according to any one of Claims 1 to 3, characterised in that the oxygen pressure is kept between 1 and 100 bars.

5. Process according to any one of Claims 1 to 4, characterised in that the reaction is catalysed by the corresponding enzyme in immobilised form.

6. Process according to any one of Claims 1 to 5, applied to the oxidation of glucose to gluconic acid with the aid of glucose oxidase.

7. Process according to Claim 6, characterised in that the reaction is carried out at a temperature of between 0 and 60°C.

8. Process according to Claim 7, characterised in that the reaction is carried out at a pH of between 4.2 and 8.

9. Process according to any one of Claims 6 to 8, applied to the selective oxidation of glucose in mixtures containing glucose and fructose.

FIG 1

## FIG 2